(19) Europäisches Patentamt
European Patent Office
Office européen des brevets

(11) Publication number: 0 000 972 A2

(12) # EUROPEAN PATENT APPLICATION

(21) Application number: 78200153.1

(22) Date of filing: 23.08.78

(51) Int. Cl.²: C 07 D 209/12, A 61 K 31/40

(30) Priority: 29.08.77 US 828355

(43) Date of publication of application: 07.03.79 Bulletin 79/5

(84) Designated Contracting States: BE CH DE FR GB NL SE

(71) Applicant: SCHERICO LTD., Töpferstrasse 5, CH-6004 Lucerne (CH)

(72) Inventor: Steinman, Martin, 46 Glendale Avenue, Livingston New Jersey 07039 (US)
Inventor: Tahbaz, Pirouz, 96 Oak Drive, Cedar Grove New Jersey 07009 (US)

(74) Representative: Antony, Fritz, Dr. et al, P.O. Box 601 Winkelriedstrasse 35, CH-6002 Lucerne (CH)

(54) **2-Substituted-3-phenylindoles, their preparation, and pharmaceutical compositions containing them.**

(57) The invention relates to novel 3-phenyl-indoles of the formula

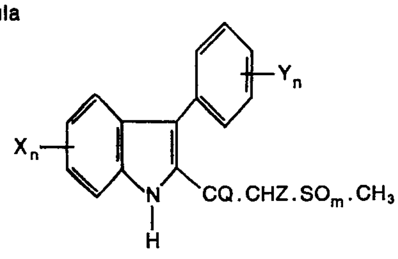

wherein X and Y are independently hydrogen or halogen atoms;

each n is 1, 2 or 3,

Q is an oxygen atom or the group (H,OH),

Z is a hydrogen atom, or provided that Q is an oxygen atom, Z can also be the group OR, wherein R is a hydrogen atom or a lower alkyl or loweralkoxy-loweralkyl group, "lower" indicating groups with 1–6 carbon atoms,

m is 1 or 2 when Z is a hydrogen atom and m is O when Z is the group OR,

to their preparation, and to pharmaceutical compositions containing them. These compounds have immunosuppressant activity and can be used for example in the treatment of rheumatoid arthritis.

EP 0 000 972 A2

"2-SUBSTITUTED-3-PHENYLINDOLES, THEIR PREPARATION, AND PHARMACEUTICAL COMPOSITIONS CONTAINING THEM."

This invention relates to novel 2-substituted-3-phenylindoles, their preparation, their use as immuno-suppressants, and to pharmaceutical compositions containing them.

According to the invention we provide novel 3-phenylindoles of the formula

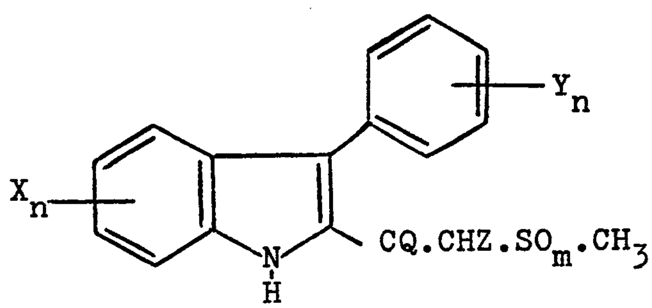

I

wherein X and Y are independently hydrogen or halogen atoms,

each n is 1, 2 or 3,

Q is an oxygen atom or the group (H,OH),

Z is a hydrogen atom or, provided that Q is an oxygen atom, Z can also be the group OR, wherein R is a hydrogen atom or a lower alkyl or loweralkoxy-loweralkyl group, "lower" indicating groups with 1 to 6 carbon atoms,

m is 1 or 2 when Z is a hydrogen atom and m is 0 when Z is the group OR.

The term "halogen" comprises fluorine, chlorine, bromine and iodine. Halogen substituents in the fused benzene ring may be in any of the 4-, 5-, 6- and 7-positions; however, monosubstitution is preferably at the 5- or 6-position whereas polysubstitution is preferably at the 4,5- or 5,6- or 4,5,6-positions. Monosubstitution of the 3-phenyl group is preferably at the 2-position whereas polysubstitution is preferably 2,6-disubstitution. At least one of X and Y is preferably halogen; $X_n$ may conveniently represent one, two or three halogen atoms, especially chlorine and/or bromine. It is particularly preferred for $X_n$ to represent two halogen atoms while Y or $Y_n$ represents a hydrogen atom or one halogen atom, especially fluorine, respectively.

The 2-substituent of the indole nucleus is preferably a methylsulfonylacetyl or especially a methylsulfinylacetyl

group; i.e. Q is an oxygen atom, Z is a hydrogen atom and m is 2 or especially 1. The 2-substituent can for example also be a methylthio-hydroxyacetyl, methylthio-loweralkoxyacetyl (especially methylthio-methoxyacetyl), 2-methylsulfinyl-1-hydroxyethyl or 2-methylsulfonyl-1-hydroxyethyl group.

Preferred compounds according to the invention include

6-bromo-5-chloro-3-(2-fluorophenyl)-2-[(methylsulfinyl)acetyl]indole,

5,6-dichloro-2-[(methylsulfinyl)acetyl]-3-(3,4-dichloromethyl)indole,

6-bromo-5-chloro-2-[(methylsulfonyl)acetyl]-3-phenylindole,

5-chloro-2-[(methylsulfinyl)acetyl]-3-(2-fluorophenyl)indole,

5,6-dichloro-2-[(methylsulfinyl)acetyl]-3-phenylindole,

2-[(methylsulfinyl)acetyl]-3-(2-fluorophenyl)indole,

4,5,6-trichloro-2-[(methylsulfinyl)acetyl]-3-phenylindole,

5-chloro-2-[(methylsulfinyl)acetyl]-3-(2,6-difluorophenyl)indole, and

5-chloro-2-[(methylsulfinyl)acetyl]-3-(2,4-dichlorophenyl)indole.

A particularly preferred compound according to the invention is 6-bromo-5-chloro-2-[(methylsulfinyl)acetyl]-3-phenylindole.

The invention further provides a process for the preparation of a compound of the formula I defined above, which comprises reacting an appropriate reactive derivative of an acid of the formula

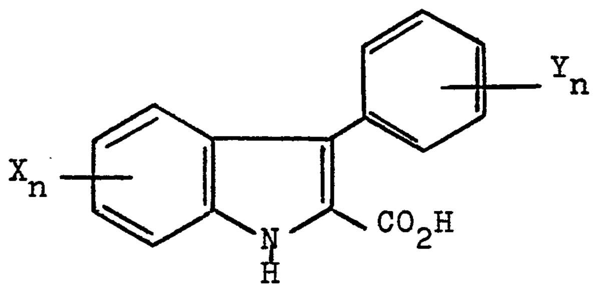

II

wherein X, Y and $\underline{n}$ are as defined in claim 1 with an anion of the formula

$$^{\ominus}CH_2.SO_m.CH_3 \qquad III$$

wherein $\underline{m}$ is 1 or 2,

in the presence of an anhydrous organic solvent and under an inert atmosphere,

whereafter, for the preparation of a compound wherein $\underline{m}$ is 2 when $\underline{m}$ in the product is $\underline{1}$, the product is oxidised,

and/or, for the preparation of a compound wherein Q is the group (H,OH), the product is reduced at the carbonyl group,

or, for the preparation of a compound wherein Z is the group OR wherein R is as defined above, a product wherein *m* is 1 is subjected to the action of acid in the presence of a solvent comprising the compound ROH.

The reactive derivative of the acid of the formula II is preferably an ester, especially a lower alkyl ester (the lower alkyl group having 1 to 6, preferably 1 to 4, carbon atoms), more especially the methyl or ethyl ester. The reactive derivative may also be the symmetrical anhydride of the acid or an N,N-disubstituted amide of the acid, e.g., an N,N-diloweralkylamide (wherein each lower alkyl group has 1 to 6, preferably 1 to 4, carbon atoms) or an N,N-pentamethylene amide.

The anion of the formula III can be prepared from dimethylsulfoxide or dimethylsulfone and a strong base, for example an alkali metal hydride, especially sodium hydride, an alkyl lithium, especially n-butyl-lithium, or potassium t-butoxide, by heating at a moderately elevated temperature, e.g., $65^{o}$ to $75^{o}$ C. for about two hours. If desired, an inert organic diluent or solvent, such as an aromatic

hydrocarbon, especially benzene, may be present. Preparation of the anion of the formula III and its reaction with the derivative of the acid of the formula II should take place in an anhydrous reaction medium and under an inert atmosphere e.g. nitrogen.

The reaction between the anion of the formula III and the derivative of the acid of the formula II is preferably carried out by stirring the reactants at about room temperature until reaction is complete (generally about 1 to 2 hours). The anion is preferably present in excess, e.g about 3 equivalents in excess relative to the derivative of the acid of the formula II. After the reaction is complete, the reaction mixture is quenched with water and then acidified and the desired product is isolated for example by filtration and recrystallisation.

A product of this process wherein $\underline{m}$ is 2 can also be prepared by oxidation of a corresponding product wherein $\underline{m}$ is 1. The oxidation is preferably effected by means of a peroxidic compound in an inert solvent, preferably an organic peracid in a halogenated organic solvent, e.g., a perbenzoic acid, especially $\underline{m}$-chloroperbenzoic acid, in chloroform, or hydrogen peroxide in acetic acid.

A product of these processes can be preferentially reduced

at the carbonyl group CQ (wherein Q is an oxygen atom) to yield a product wherein Q is the group (H, OH), without reduction at the sulfinyl or sulfonyl group. This reduction is preferably effected by means of a borohydride, especially sodium borohydride, in a water-miscible inert organic solvent which may contain water, e.g. water-miscible ethers such as aqueous tetrahydrofuran or dioxan or lower alkanols, especially methanol or ethanol.

A product wherein m is 1 may be subjected to a Pummerer-type rearrangement in the presence of acid, preferably mineral acid, and a compound ROH (wherein R is as defined above) to yield a compound of the formula I wherein m is 0 and Z is OR (wherein R is as defined above). The mineral acid is conveniently hydrochloric acid. When this reaction is carried out in the presence of water, the product is a 2-[(methylthio)(hydroxy)acetyl]-3-phenylindole (which may be called a 3-phenylindole-2-glyoxal methyl hemimercaptal); when this reaction is carried out in the presence of a lower alkanol (or loweralkoxy-loweralkanol), the product is a 2-[(methylthio)loweralkoxy or loweralkoxy-loweralkoxy)acetyl-3-phenylindole.

The derivatives, e.g. the lower alkyl esters, of the 3-phenylindole-2-carboxylic acids of the formula II either are

known compounds or can be prepared by known methods. Thus an appropriately $X_n$-substituted or unsubstituted phenylhydrazine hydrogen halide can be reacted with an appropriately $Y_n$-substituted or unsubstituted β-phenylpyruvic acid (usually as an alkali metal salt) to produce the corresponding phenylhydrazone of the β-phenylpyruvic acid, which is cyclized under conditions usual in the Fischer indole synthesis, i.e., using an acid catalyst e.g. a mineral acid such as sulfuric acid or a Lewis acid such as zinc chloride. If the phenylhydrazone of the β-phenylpyruvic acid is cyclised by means of concentrated sulfuric acid in a lower alkanol, the product is the corresponding alkyl 3-phenylindole-2-carboxylate. This reaction often yields isomeric products, which can be separated by chromatography.For example, the cyclization of β-phenylpyruvic acid 3,4-dichlorophenylhydrazone by heating with concentrated sulfuric acid in ethanol will produce a mixture containing ethyl 5,6-dichloro-3-phenylindole-2-carboxylate and ethyl 4,5-dichloro-3-phenylindole-2-carboxylate. The mixture is chromatographed on silica gel in chloroform and the isomeric products are easily separated.

The following preparations show how the starting materials for the process according to the invention can be obtained:

## Preparation 1

### Phenylpyruvic acid 3,4-dichlorophenylhydrazone

Add 21.0 g. of sodium β-phenylpyruvate monohydrate and 21.3 g. of 3,4-dichlorophenylhydrazine hydrochloride to 300 ml. of ethanol and reflux the resulting mixture for 30 minutes. Cool, add water, and collect and dry the solid to yield phenylpyruvic acid 3,4-dichlorophenylhydrazone, m.p. 166-167°C.

## Preparation 2

### Ethyl 5,6-dichloro-3-phenylindole-2-carboxylate and ethyl 4,5-dichloro-3-phenylindole-2-carboxylate

Add 100 ml. of concentrated sulfuric acid to a solution of 20.0 g. of phenylpyruvic acid 3,4-dichlorophenylhydrazone in 600 ml. of ethanol. Reflux the mixture for 2 1/2 hours, cool, add water, and collect and dry the solid. Chromatograph this on silica gel with chloroform to obtain ethyl 5,6-dichloro-3-phenylindole-2-carboxylate, m.p. 201-202°C. Later fractions yield the isomeric ethyl 4,5-dichloro-3-phenylindole-2-carboxylate.

## Preparation 3

Ethyl 6-bromo-5-chloro-3-(2-fluorophenyl)indole-2-carboxylate

Add 1.0 ml. of bromine to 4.6 g. of ethyl 5-chloro-3-(2-fluorophenyl)indole-2-carboxylate in 250 ml. of glacial acetic acid. Stir for 16 hours at room temperature, add water, collect the precipitate and wash it with warm methanol. Collect and dry the ethyl 6-bromo-5-chloro-3-(2-fluorophenyl)indole-2-carboxylate, m.p. 193-194°C.

Thus, by following the teachings of Preparations 1-3, with the use of the appropriate starting materials, the following compounds may be produced:

ethyl 5-chloro-3-phenylindole-2-carboxylate,

ethyl 6-bromo-5-chloro-3-phenylindole-2-carboxylate,

ethyl 5-chloro-3-(2-fluorophenyl)indole-2-carboxylate,

ethyl 4,5,6-trichloro-3-phenylindole-2-carboxylate,

ethyl 5,6-dichloro-3-phenylindole-2-carboxylate,

ethyl 4,5-dichloro-3-phenylindole-2-carboxylate,

ethyl 5-chloro-3-(4-fluorophenyl)indole-2-carboxylate,

ethyl 6-bromo-5-chloro-3-(4-fluorophenyl)indole-2-carboxylate,

ethyl 5,6-dichloro-3-(3,4-dichlorophenyl)indole-2-carboxylate,

ethyl 5-chloro-3-(4-chlorophenyl)indole-2-carboxylate,

ethyl 6-chloro-3-phenylindole-2-carboxylate,

ethyl 5-bromo-6-chloro-3-(3-bromophenyl)indole-2-carboxylate,

ethyl 4,6-dibromo-3-phenylindole-2-carboxylate,

ethyl 5-chloro-3-(2,4-dichlorophenyl)indole-2-carboxylate,

ethyl 5-chloro-3-(2,6-difluorophenyl)indole-2-carboxylate,

ethyl 5-bromo-6-fluoro-3-phenylindole-2-carboxylate,

ethyl 5,6-difluoro-3-phenylindole-2-carboxylate,

ethyl 5,6-difluoro-3-(4-fluorophenyl)indole-2-carboxylate,

ethyl 5,6-difluoro-3-(3,4-difluorophenyl)indole-2-carboxylate,

ethyl 6-chloro-3-(4-chlorophenyl)indole-2-carboxylate,

ethyl 5-bromo-6-chloro-3-phenylindole-2-carboxylate and

ethyl 5-chloro-3-(2,6-dichlorophenyl)indole-2-carboxylate.

The following Examples illustrate the invention:

## Example 1

### 6-Bromo-5-chloro-2[(methylsulfinyl)acetyl]-3-phenylindole

Stir a 57% sodium hydride dispersion (13 g.) in dimethylsulfoxide (100 ml.) and benzene (100 ml.) and heat at 65-75° C. for 2 hours under nitrogen. Add ethyl 6-bromo-5-chloro-3-phenylindole-2-carboxylate (30 g.) in dimethylsulfoxide (50 ml.) and benzene (30 ml.) at 15-20°C., and stir the

mixture at room temperature for one hour. Cool the resulting mixture to 15°C., add 50 ml. of water very slowly and then an additional 550 ml. of water. Add 25 ml. of acetic acid, stir and then filter. Wash the resulting solid with water and then hexane. Recrystallize the desired product from methanol-methylene chloride to obtain 6-bromo-5-chloro-2[(methylsulfinyl)acetyl]-3-phenylindole, m.p. 169-170°C.

By replacing the ethyl 5-chloro-6-bromo-3-phenylindole-2-carboxylate with the product of preparation 3 or with the compounds listed after Preparation 3 and by substantially following the method of this Example, the following compounds are produced:

6-bromo-5-chloro-2-[methylsulfinyl)acetyl]-3-(2-fluorophenyl) indole,

5-chloro-2[(methylsulfinyl)acetyl]-3-phenylindole,

5-chloro-2[(methylsulfinyl)acetyl]-3-(2-fluorophenyl)indole,

4,5,6-trichloro-2[(methylsulfinyl)acetyl]-3-phenylindole,

5,6-dichloro-2[(methylsulfinyl)acetyl]-3-phenylindole,

4,5-dichloro-2[(methylsulfinyl)acetyl]-3-phenylindole,

5-chloro-2[(methylsulfinyl)acetyl]-3-(4-fluorophenyl)indole,

6-bromo-5-chloro-2[(methylsulfinyl)acetyl]-3-(4-fluorophenyl) indole,

5,6-dichloro-2-[(methylsulfinyl)acetyl]-3-(3,4-dichlorophenyl)indole,

5-chloro-2[(methylsulfinyl)acetyl]-3-(4-chlorophenyl)indole,
6-chloro-2[(methylsulfinyl)acetyl]-3-phenylindole,
5-bromo-6-chloro-2[(methylsulfinyl)acetyl]-3-(3-bromophenyl)indole,
4,6-dibromo-2[(methylsulfinyl)acetyl]-3-phenylindole,
5-chloro-2[(methylsulfinyl)acetyl]-3-(2,4-dichlorophenyl)indole,
5-chloro-2[(methylsulfinyl)acetyl]-3-(2,6-difluorophenyl)indole,
5-bromo-6-fluoro-2[(methylsulfinyl)acetyl]-3-phenylindole,
5,6-difluoro-2[(methylsulfinyl)acetyl]-3-phenylindole,
5,6-difluoro-2[(methylsulfinyl)acetyl]-3-(4-fluorophenyl)indole,
5,6-difluoro-2[(methylsulfinyl)acetyl]-3-(3,4-difluorophenyl)indole,
6-chloro-2[(methylsulfinyl)acetyl]-3-(4-chlorophenyl)indole,
5-bromo-6-chloro-2[(methylsulfinyl)acetyl]-3-phenylindole and
5-chloro-2[(methylsulfinyl)acetyl]-3-(2,6-dichlorophenyl)indole.

Example 2

6-Bromo-5-chloro-2[(methylsulfonyl)acetyl]-3-phenylindole

Stir a mixture of 6.0 g of 6-bromo-5-chloro-2[(methylsulfinyl)acetyl]-3-phenylindole and 6.0 g of m-chloroperbenzoic

acid in 150 ml. of chloroform at room temperature for 2 hours. Concentrate the mixture to about 50 ml. *in vacuo*, filter off the product and wash it with 20 ml. of cold chloroform to obtain 6-bromo-5-chloro-2[(methylsulfonyl)acetyl]-3-phenylindole, m.p. 224-225° C.

By replacing the 6-bromo-5-chloro-2[(methylsulfinyl)acetyl]-3-phenylindole with the compounds obtained by the method of Example 1 and by substantially following the method of this Example, the following compounds are produced:

6-bromo-5-chloro-2[(methylsulfonyl)acetyl]-3-(2-fluorophenyl)indole,

5-chloro-2[(methylsulfonyl)acetyl]-3-phenylindole,

5-chloro-2[(methylsulfonyl)acetyl]-3-(2-fluorophenyl)indole,

4,5,6-trichloro-2[(methylsulfonyl)acetyl]-3-phenylindole,

5,6-dichloro-2[(methylsulfonyl)acetyl]-3-phenylindole,

4,5-dichloro-2[(methylsulfonyl)acetyl]-3-phenylindole,

5-chloro-2[(methylsulfonyl)acetyl]-3-(4-fluorophenyl)indole,

6-bromo-5-chloro-2[(methylsulfonyl)acetyl]-3-(4-fluorophenyl)indole,

5,6-dichloro-2[(methylsulfonyl)acetyl]-3-(3,4-dichlorophenyl)indole,

5-chloro-2[(methylsulfonyl)acetyl]-3-(4-chlorophenyl)indole,

6-chloro-2[(methylsulfonyl)acetyl]-3-phenylindole,

5-bromo-6-chloro-2[(methylsulfonyl)acetyl]-3-(3-bromophenyl)indole,

4,6-dibromo-2[(methylsulfonyl)acetyl]-3-phenylindole,

5-chloro-2[(methylsulfonyl)acetyl]-3-(2,4-dichlorophenyl) indole,

5-chloro-2[(methylsulfonyl)acetyl]-3-(2,6-difluorophenyl) indole,

5-bromo-6-fluoro-2[(methylsulfonyl)acetyl]-3-phenylindole,

5,6-difluoro-2[(methylsulfonyl)acetyl]-3-phenylindole,

5,6-difluoro-2[(methylsulfonyl)acetyl]-3-(4-fluorophenyl) indole,

5,6-difluoro-2[(methylsulfonyl)acetyl]-3-(3,4-difluorophenyl) indole,

6-chloro-2[(methylsulfonyl)acetyl]-3-(4-chlorophenyl)indole,

5-bromo-6-chloro-2[(methylsulfonyl)acetyl]-3-phenylindole and

5-chloro-2[(methylsulfonyl)acetyl]-3-(2,6-dichlorophenyl) indole.

## Example 3

### 6-Bromo-5-chloro-2[(1-hydroxy-2-methylsulfinyl)ethyl]-3--phenylindole

To 6-bromo-5-chloro-2[(methylsulfinyl)acetyl]-3-phenylindole (2.5 g.) in 50 ml. of ethanol add 0.3 g. of sodium borohydride and, after a solution is obtained (about 10 minutes) stir the mixture for another 30 minutes. Slowly add 1 ml.

of acetic acid, and then add 100 ml. of water. Decant the liquid from the insoluble material, dissolve it in ethanol and add ether-hexane to obtain 6-bromo-5-chloro-2[(1-hydroxy--2-methylsulfinyl)ethyl]-3-phenylindole, m.p. 145-148°C.

By replacing the 6-bromo-5-chloro-2[(methylsulfinyl)acetyl]--3-phenylindole with the compounds obtained by the method of Example 1, and by substantially following the method of this Example, the following compounds are produced:

6-bromo-5-chloro-2[(1-hydroxy-2-methylsulfinyl)ethyl]-3-(2-fluorophenyl)indole,

5-chloro-2[(1-hydroxy-2-methylsulfinyl)ethyl]-3-phenylindole,

5-chloro-2[(1-hydroxy-2-methylsulfinyl)ethyl]-3-[(2-fluorophenyl)indole,

4,5,6-trichloro-2[(1-hydroxy-2-methylsulfinyl)ethyl]-3-phenylindole,

5,6-dichloro-2[1-hydroxy-2-methylsulfinyl)ethyl]-3-phenylindole,

4,5-dichloro-2[(1-hydroxy-2-methylsulfinyl)ethyl]-3-phenylindole,

5-chloro-2[(1-hydroxy-2-methylsulfinyl)ethyl]-3-(4-fluorophenyl)indole,

6-bromo-5-chloro-2[1-hydroxy-2-methylsulfinyl)ethyl]-3-(4--fluorophenyl)indole,

5,6-dichloro-2[(1-hydroxy-2-methylsulfinyl)ethyl]-3-(3,4--dichlorophenyl)indole,

5-chloro-2[(1-hydroxy-2-methylsulfinyl)ethyl]-3-(4-chlorophenyl)indole,

6-chloro-2[(1-hydroxy-2-methylsulfinyl)ethyl]-3-phenylindole,

5-bromo-6-chloro-2[(1-hydroxy-2-methylsulfinyl)ethyl]-3-(3-bromophenyl)indole,

4,6-dibromo-2[(1-hydroxy-2-methylsulfinyl)ethyl]-3-phenylindole,

5-chloro-2[(1-hydroxy-2-methylsulfinyl)ethyl]-3-(2,4-dichlorophenyl)indole,

5-chloro-2[(1-hydroxy-2-methylsulfinyl)ethyl]-3-(2,6-difluorophenyl)indole,

5-bromo-6-fluoro-2[(1-hydroxy-2-methylsulfinyl)ethyl]-3-phenylindole,

5,6-difluoro-2[(1-hydroxy-2-methylsulfinyl)ethyl]-3-phenylindole,

5,6-difluoro-2[1-hydroxy-2-methylsulfinyl)ethyl]-3-(4-fluorophenyl)indole,

5,6-difluoro-2[(1-hydroxy-2-methylsulfinyl)ethyl]-3-(3,4-difluorophenyl)indole,

6-chloro-2[(1-hydroxy-2-methylsulfinyl)ethyl]-3-(4-chlorophenyl)indole,

5-bromo-6-chloro-2[(1-hydroxy-2-methylsulfinyl)ethyl]-3-phenylindole and

5-chloro-2[(1-hydroxy-2-methylsulfinyl)ethyl]-3-(2,6-dichlorophenyl)indole.

## Example 4

6-Bromo-5-chloro-2-[(1-hydroxy-2-methylsulfonyl)ethyl]-3-phenylindole

To 6-bromo-5-chloro-2[(methylsulfonyl)acetyl]-3-phenylindole (2.2 g.) in 75 ml. of ethanol add 0.175 g. of sodium borohydride. After a solution is obtained (about 10 minutes) stir the mixture for another 30 minutes, then slowly add 2 ml. of acetic acid. Treat with charcoal, filter, and add 100 ml. of water to the filtrate to obtain 6-bromo-5-chloro-2-[(1-hydroxy-2-methylsulfonyl)ethyl]-3-phenylindole, m.p. 187-188°C.

Similarly, by replacing the 6-bromo-5-chloro-2[(2-methylsulfonyl)acetyl]-3-phenylindole with the compounds obtained by the method of Example 2, and by substantially following the method of this Example, the following compounds are produced:

6-bromo-5-chloro-2[(1-hydroxy-2-methylsulfonyl)ethyl]-3-(2-fluorophenyl)indole,

5-chloro-2[(1-hydroxy-2-methylsulfonyl)ethyl]-3-phenylindole,

5-chloro-2[(1-hydroxy-2-methylsulfonyl)ethyl]-3-(2-fluorophenyl)indole,

4,5,6-trichloro-2[(1-hydroxy-2-methylsulfonyl)ethyl]-3-phenylindole,

5,6-dichloro-2[(1-hydroxy-2-methylsulfonyl)ethyl]-3-phenyl-indole,

4,5-dichloro-2[(1-hydroxy-2-methylsulfonyl)ethyl]-3-phenyl-indole,

5-chloro-2[(1-hydroxy-2-methylsulfonyl)ethyl]-3-(4-fluorophenyl)indole,

6-bromo-5-chloro-2[(1-hydroxy-2-methylsulfonyl)ethyl]-3-(4-fluorophenyl)indole,

5,6-dichloro-2[(1-hydroxy-2-methylsulfonyl)ethyl]-3-(3,4-dichlorophenyl) indole,

5-chloro-2[(1-hydroxy-2-methylsulfonyl)ethyl]-3-(4-chlorophenyl)indole,

6-chloro-2[(1-hydroxy-2-methylsulfonyl)ethyl]-3-phenylin-dole,

5-bromo-6-chloro-2[(1-hydroxy-2-methylsulfonyl)ethyl]-3-(3-bromophenyl)indole,

4,6-dibromo-2[(1-hydroxy-2-methylsulfonyl)ethyl]-3-phenyl-indole,

5-chloro-2[(1-hydroxy-2-methylsulfonyl)ethyl]-3-(2,4-dichlorophenyl)indole,

5-chloro-2[(1-hydroxy-2-methylsulfonyl)ethyl]-3-(2,6-difluorophenyl)indole,

5-bromo-6-fluoro-2[(1-hydroxy-2-methylsulfonyl)ethyl]-3-phenylindole,

5,6-difluoro-2[(1-hydroxy-2-methylsulfonyl)ethyl]-3-phe-nylindole,

5,6-difluoro-2[(1-hydroxy-2-methylsulfonyl)ethyl]-3-(4-fluorophenyl)indole,

5,6-difluoro-2[(1-hydroxy-2-methylsulfonyl)ethyl]-3-(3,4-difluorophenyl)indole,

6-chloro-2[(1-hydroxy-2-methylsulfonyl)ethyl]-3-(4-chlorophenyl)indole,

5-bromo-6-chloro-2[(1-hydroxy-2-methylsulfonyl)ethyl]-3-phenylindole, and

5-chloro-2[(1-hydroxy-2-methylsulfonyl)ethyl]-3-(2,6-dichlorophenyl)indole.

## Example 5

6-Bromo-5-chloro-3-phenylindole-2-glyoxal methyl hemimercaptal

Slowly add 15 ml. of 6N hydrochloric acid to 6-bromo-5-chloro-2[(methylsulfinyl)acetyl]-3-phenylindole (2 g.) in dimethyl sulfoxide (75 ml.), stir the resulting mixture for 3 hours, quench with ice-water, and collect and dry the solid to yield 6-bromo-5-chloro-3-phenylindole-2-glyoxal methyl hemimercaptal, m.p. 153-154°C.

By replacing the 6-bromo-5-chloro-2-[(methylsulfinyl)acetyl]-3-phenylindole with the compounds obtained by the method of Example 1, and by substantially following the method

of this Example, the following compounds are produced:

6-bromo-5-chloro-3-(2-fluorophenyl)indole-2-glyoxal methyl hemimercaptal,

5-chloro-3-phenylindole-2-glyoxal methyl hemimercaptal,

5-chloro-3-(2-fluorophenyl)indole-2-glyoxal methyl hemimercaptal,

4,5,6-trichloro-3-phenylindole-2-glyoxal methyl hemimercaptal,

5,6-dichloro-3-phenylindole-2-glyoxal methyl hemimercaptal,

4,5-dichloro-3-phenylindole-2-glyoxal methyl hemimercaptal,

5-chloro-3-(4-fluorophenyl)indole-2-glyoxal methyl hemimercaptal,

6-bromo-5-chloro-3-(4-fluorophenyl)indole-2-glyoxal methyl hemimercaptal,

5,6-dichloro-3-(3,4-dichlorophenyl)indole-2-glyoxal methyl hemimercaptal,

5-chloro-3-(4-chlorophenyl)indole-2-glyoxal methyl hemimercaptal,

6-chloro-3-phenylindole-2-glyoxal methyl hemimercaptal,

5-bromo-6-chloro-3-(3-bromophenyl)indole-2-glyoxal methyl hemimercaptal,

4,6-dibromo-3-phenylindole-2-glyoxal methyl hemimercaptal,

5-chloro-3-(2,4-dichlorophenyl)indole-2-glyoxal methyl hemimercaptal,

5-chloro-3-(2,6-difluorophenyl)indole-2-glyoxal methyl hemimercaptal,

5-bromo-6-fluoro-3-phenylindole-2-glyoxal methyl hemimercaptal,

5,6-difluoro-3-phenylindole-2-glyoxal methyl hemimercaptal,

5,6-difluoro-3-(4-fluorophenyl)indole-2-glyoxal methyl hemimercaptal,

5,6-difluoro-3-(3,4-difluorophenyl)indole-2-glyoxal methyl hemimercaptal,

6-chloro-3-(4-chlorophenyl)indole-2-glyoxal methyl hemimercaptal,

5-bromo-6-chloro-3-phenylindole-2-glyoxal methyl hemimercaptal and

5-chloro-3-(2,6-dichlorophenyl)indole-2-glyoxal methyl hemimercaptal.

## Example 6

### 6-Bromo-5-chloro-2[(methylthio)(methoxy)acetyl]-3-phenylindole

Add 2 ml. of concentrated hydrochloric acid to 6-bromo-5-chloro-2[(methylsulfinyl)acetyl]-3-phenylindole (5.0 g.) in 50 ml. of methanol and 50 ml. of tetrahydrofuran and maintain the mixture at 60$^{o}$ C. for 2 hours. Cool, add water and collect and dry the resulting solid to obtain 6-bromo-5-chloro-2[(methylthio)(methoxy)acetyl]-3-phenylindole, m.p. 145-147$^{o}$C.

By replacing the 6-bromo-5-chloro-2[(methylsulfinyl)acetyl-3-phenylindole with the compounds obtained by the method of Example 1, and by substantially following the method of this Example, the following compounds are produced:

6-bromo-5-chloro-2[(methylthio)(methoxy)acetyl]-3(2-fluorophenyl)indole,

5-chloro-2[(methylthio)(methoxy)acetyl]-3-phenylindole,

5-chloro-2[(methylthio)(methoxy)acetyl]-3-(2-fluorophenyl) indole,

4,5,6-trichloro-2[(methylthio)(methoxy)acetyl]-3-phenylindole,

5,6-dichloro-2[(methylthio)(methoxy)acetyl]-3-phenylindole,

4,5-dichloro-2[(methylthio)(methoxy)acetyl]-3-phenylindole,

5-chloro-2[(methylthio)(methoxy)acetyl]-3-(4-fluorophenyl) indole,

6-bromo-5-chloro-2[(methylthio)(methoxy)acetyl]-3-(4-fluorophenyl)indole,

5,6-dichloro-2[(methylthio)(methoxy)acetyl]-3-(3,4-dichlorophenylindole,

5-chloro-2[(methylthio)(methoxy)acetyl]-3-(4-chlorophenyl) indole,

6-chloro-2[(methylthio)(methoxy)acetyl]-3-phenylindole,

5-bromo-6-chloro-2[(methylthio)(methoxy)acetyl]-3-(3-bromophenyl)indole,

4,6-dibromo-2[(methylthio)(methoxy)acetyl]-3-phenylindole,

5-chloro-2[(methylthio)(methoxy)acetyl]-3-(2,4-dichlorophenyl)indole,

5-chloro-2[(methylthio)(methoxy)acetyl]-3-(2,6-difluorophenyl)indole,

5-bromo-6-fluoro-2[(methylthio)(methoxy)acetyl]-3-phenylindole,

5,6-difluoro-2[(methylthio)(methoxy)acetyl]-3-phenylindole,

5,6-difluoro-2[(methylthio)(methoxy)acetyl]-3-(4-fluorophenyl)indole,

5,6-difluoro-2[(methylthio)(methoxy)acetyl]-3-(3,4-difluorophenyl)indole,

6-chloro-2[(methylthio)(methoxy)acetyl]-3-(4-chlorophenyl) indole,

5-bromo-6-chloro-2[(methylthio)(methoxy)acetyl]-3-phenylindole and

5-chloro-2[(methylthio)(methoxy)acetyl]-3-(2,6-dichlorophenyl) indole.

Similarly, by replacing the methanol of this Example with equivalent quantities of straight and branched-chain lower alcohols having up to six carbon atoms, the corresponding 2-[(methylthio) (loweralkoxy)acetyl]-3-phenylindoles are produced.

The compounds of this invention inhibit antibody immune

reactions and cell-mediated immune reactions. The antibody immune reactions include the immune response to sheep erythrocytes and the immune responses to trinitrophenylated liposaccharide in mice as assessed by the spleen assay of Jerne et al., "Cell-bound Antibodies" Wistar Institute Press, 1963. Immune reactions classified as cell-mediated, delayed type hypersensitivities, include the late secondary migratory lesions in rats injected with Freund's adjuvant (in accordance with the techniques described in British J. Pharmacology, 21 : 127-136 and 24 : 632-640), skin transplant rejection in mice and rats and mammary gland rejection (as described in "Transplantation of Cells and Tissues", Wistar Institute Press, 1961), contact and protein hypersensitivities in guinea pigs, rabbits and rats (Uhr, Physiology Review, 46 : 359-419) and experimental allergic encephalomyelitis in rats. From these tests and comparisons with known immunosuppressants, it may be determined that the compounds are effective in suppressing immune responses at about 1 to 100 mg./kg. mammalian body weight. Disease states against which the immunosuppressant activity of the compounds of this invention are useful include rheumatoid arthritis, ulcerative colitis, allergies, systemic lupus erythematosus, hemalytic anemia and Crohn's disease. In

their use as immunosuppressants the compounds have low toxicity and in particular are substantially non-cytotoxic at therapeutic doses.

In their use, the compounds of this invention may be combined with inert pharmaceutical carriers or excipients such as lactose, mannitol and starch. For parenteral injection, the compounds may be formulated with an inert, parenterally acceptable vehicle, such as water, saline or sesame oil. These formulations may be compounded according to methods well known to those skilled in the pharmaceutical art. Preferably the compounds are administered in 3-4 daily doses although the specific regimen will be dependent upon the severity and nature of the particular disease state.

The invention therefore provides pharmaceutical compositions comprising, as active ingredient, at least one compound of the formula I defined above in association with a suitable carrier or excipient.

The compounds of the formula I can be administered in the form of dosage units, in particular shaped dosage units such as tablets, capsules and suppositories. Dosage units conveniently contain from about 5 to about 500 mg., preferably 20 to 100 mg., of active compound of the formula I.

Example of a Pharmaceutical Preparation: Tablets

| Ingredient | For 10,000 tablets |
|---|---|
| Active ingredient of formula I | 250.0 g. |
| Lactose | 1000.0 g. |
| Corn starch | 680.0 g. |
| Corn starch as 10% paste | 50.0 g. |
| Magnesium stearate | 20.0 g. |

Mix the active ingredient, lactose and 600 g. of the corn starch, and pass through a pulverizing mill if necessary. Granulate the so-obtained mixture with the starch paste, adding additional water if necessary to make a damp granulation. Pass the granulation through an impact mill to produce 8-12 mesh granules. Spread the granulation on trays and dry in a draft-oven at 35-40°C. Reduce the dried granulation to 16-24 mesh size and blend it with the remaining 80.0 g. of cornstarch and with the magnesium stearate until a uniform mixture is obtained. Compress to 200 mg. tablets containing 25 mg. of active ingredient.

In this Example, the active ingredient is preferably 6-bromo-5-chloro-2-[(methylsulfinyl)acetyl]-3-phenylindole, but may be any other compound of the formula I defined above, especially a compound named herein.

The claims defining the Invention are as follows:

1. Novel 3-phenyl-indoles of the formula

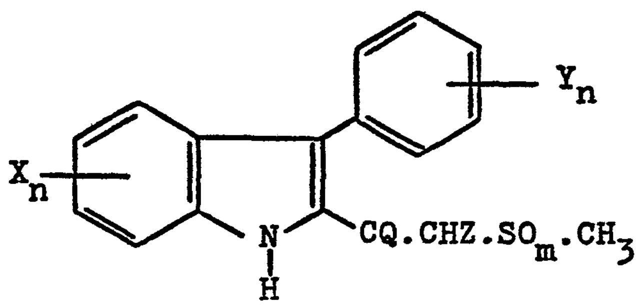

I

wherein X and Y are independently hydrogen or halogen atoms;

each n is 1,2 or 3,

Q is an oxygen atom or the group (H,OH),

Z is a hydrogen atom, or provided that Q is an oxygen atom, Z can also be the group OR, wherein R is a hydrogen atom or a lower alkyl or loweralkoxy-loweralkyl group, "lower" indicating groups with 1-6 carbon atoms,

m is 1 or 2 when Z is a hydrogen atom and m is 0 when Z is the group OR.

2. Compounds as claimed in claim 1 characterised by one or more of the following features:

a) Q is an oxygen atom, Z is a hydrogen atom and m is 1 or 2,

b) $X_n$ represents one, two or three halogen atoms,

c) $X_n$ represents two halogen atoms and Y represents a hydrogen atom,

d) $X_n$ represents two halogen atoms and $Y_n$ represents one halogen atom.

3. A compound claimed in claim 1, namely 6-bromo-5-chloro-2-[(methylsulfinyl)acetyl]-3-phenylindole.

4. Compounds as claimed in claim 1, namely 6-bromo-5-chloro-3-(2-fluorophenyl)-2-[(methylsulfinyl)acetyl] indole,

5,6-dichloro-2-[(methylsulfinyl)acetyl]-3-(3,4-dichlorophenyl)indole,

6-bromo-5-chloro-2-[(methylsulfonyl)-acetyl]-3-phenylindole,

5-chloro-2-[(methylsulfinyl)-acetyl]-3-(2-fluorophenyl)indole,

5,6-dichloro-2-[(methylsulfinyl)acetyl]-3-phenylindole,

2-[(methylsulfinyl)acetyl]-3-(2-fluorophenyl)indole,

4,5,6-trichloro-2-[(methylsulfinyl)acetyl]-3-phenylindole,

5-chloro-2-[(methylsulfinyl)acetyl]-3-(2,6-difluorophenyl)indole, and

5-chloro-2-[(methylsulfinyl)acetyl]-3-(2,4-dichlorophenyl)indole.

5. Process for the preparation of a compound claimed in claim 1, which comprises reacting a reactive derivative of an acid of the formula

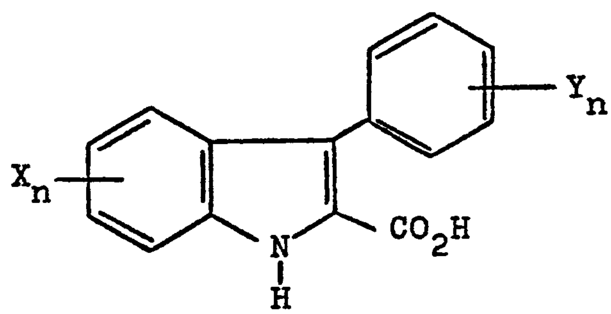

II

wherein X, Y and n are as defined in claim 1 with an anion of the formula

$$^{\ominus}CH_2.SO_m.CH_3 \qquad III$$

wherein m is 1 or 2, in the presence of an anhydrous organic solvent and under an inert atmosphere, whereafter, for the preparation of a compound wherein m is 2 when m in the product is 1, the product is oxidised, and/or for the preparation of a compound wherein Q is

the group (H,OH), the product is reduced at the carbonyl group,
or, for the preparation of a compound wherein Z is the group OR wherein R is as defined in claim 1, a product wherein m is 1 is subjected to the action of acid in the presence of a solvent comprising the compound ROH.

6. A process as claimed in claim 5, wherein the reactive derivative is a lower alkyl ester, preferably an ethyl ester, and/or wherein the anion is present in excess, preferably in about 3 equivalents excess.

7. Pharmaceutical compositions containing as active ingredient at least one compound as claimed in any of claims 1 to 4 together with pharmaceutical carrier or excipient.

8. Compositions as claimed in claim 7 in the form of dosage units, such as tablets, capsules or suppositories, especially dosage units containing from 5 to 500 mg.of active ingredient, preferably from 20 to 100 mg. of active ingredient.

9. Compositions as claimed in claim 7 in the form of injectable compositions.

10. Compositions as claimed in any of claims 7 to 9 wherein the active ingredient is 6-bromo-5-chloro-2-[(methylsulfinyl)acetyl]-3-phenylindole.